# EUROPEAN PATENT SPECIFICATION

(11) **EP 0 668 775 B1**
(45) Date of publication and mention of the grant of the patent: **04.02.1998**
(21) Application number: 93923561.0
(22) Date of filing: 27.10.1993
(51) Int. Cl.: A61K 38/24, G01N 33/76

(54) **PURIFIED TSH PREPARATION, PROCESS FOR ITS PRODUCTION AND ITS USE FOR THE PRODUCTION OF TSH TRACERS FOR TSH RECEPTOR ASSAYS AND IN TSH RECEPTOR ASSAYS**
REINE TSH ZUBEREITUNG, VERFAHREN ZUR HERSTELLUNG UND IHRE ANWENDUNG FÜR DIE HERSTELLUNG VON TSH SPÜRELEMENTEN FÜR TSH REZEPTOR NACHWEIS UND IN TSH REZEPTOR TESTPROBEN
PREPARATION DE THYROTROPINE PURIFIEE, PROCEDE DE PRODUCTION ET UTILISATION POUR LA PRODUCTION DE TRACEURS DE THYROTROPINE POUR ET DANS LES DOSAGES DE RECEPTEURS DE LA THYROTROPINE

(30) Priority: 05.11.1992 DE 4237430
(43) Date of publication of application: 30.08.1995
(73) Proprietor: B.R.A.H.M.S Diagnostica GmbH, D-12099 Berlin (DE)
(72) Inventor: BERGMANN, Andreas, D-12351 Berlin (DE); STRUCK, Joachim, D-12247 Berlin (DE)
(74) Representative: Andrae, Steffen, Dr.
(86) International application number: EP9302980
(87) International publication number: WO9409814

(56) References cited:
- WO-A-83/04372
- GB-A- 2 173 803
- US-A- 4 357 310
- ENDOCRINOLOGY vol. 111, no. 1 , January 1988 pages 319 - 324 ARJAN J. LOCK ET AL. 'PURIFICATION OF BOVINE THYROID-STIMULATING HORMONE BY A MONOCLONAL ANTIBODY'
- DATABASE WPI Section Ch, Week 9221, Derwent Publications Ltd., London, GB; Class B04, AN 92-171661 & JP,A,4 108 397 (KOKUSAI SHIYAKU KK) 9 April 1992

## Description

The present invention relates to a process for the production of a radioiodinated TSH tracer on the basis of a TSH preparation which is obtained from crude bovine TSH (thyrotropin) by purification, and to the use of said radioiodinated TSH tracer in a TSH radioreceptor assay for the determination of TSH receptor autoantibodies.

TSH is a pituitary hormone which performs a central task in the regulation of the thyroid function. Its release is stimulated by the hormone TRH formed in the hypothalamus and controls the formation and release of the most important thyroid hormone thyroxine (T4). By means of a feedback, the content of thyroxine in the serum controls the release of TSH. The formation of thyroxine by the thyroid cells is stimulated by TSH by virtue of the fact that the TSH released by the pituitary binds to the TSH receptor of the thyroid cell membrane.

However, various types of autoantibodies can also be formed against the same TSH receptor in different pathogenic conditions. Depending on the type of the autoantibodies, either the formation and release of thyroxine may be inhibited by shielding of the TSH molecules at the TSH receptors or this thyroid hormone is released in an uncontrolled manner because the TSH receptor autoantibodies, like the TSH, stimulate the synthesis and release of thyroid hormones. The excess thyroid hormone resulting in the latter case manifests itself, inter alia, as a hyperthyroidosis such as Graves' disease.

The detection of TSH receptor autoantibodies is thus very important for clinical practice, and a number of radioreceptor assays which permit the determination of such autoantibodies in biological fluid are already known.

Such TSH receptor assays function similarly to competitive radioimmunoassays, except that TSH receptors are used as a specific binding agent in the assay instead of antibodies as in the classical radioimmunoassays. In the known TSH receptor autoantibody assays, these receptors originate from animal thyroid tissue, in particular porcine TSH receptor preparations being used. They are available in soluble form and are used in an amount which is less than the stoichiometric amount. When the assay is carried out, TSH receptor autoantibodies from a patient's serum and a labelled TSH which is used as a tracer and is usually a bovine ¹²⁵I-labelled TSH compete for a limited number of binding sites of this receptor reagent. The smaller the number of autoantibodies present in the serum sample, the more the labelled TSH tracer molecules are bound by the receptor.

The basic principles and details of the determination of the TSH receptor autoantibodies in human serum are described in a large number of publications, of which - in addition to the product information of the various manufacturers (TRAK® of the Applicant; assays of the companies Byk Sangtec, Biocode and Chronus) in connection with their assays - the following may be mentioned in particular: the article by L.C. Harrison and P.J. Leedman in Clin. Biochem. Vol. 23, pages 43 to 48, 1990, Bernard Rees Smith et al. in Endocrine Reviews, Volume 9, No. 1, pages 106 to 121, 1989, and Bernard Rees Smith and Reginald Hall, Methods in Enzymology, Volume 74, pages 405 to 420, 1981. While the TSH receptor required for the TSH receptor assay is usually obtained in a comparatively simple manner by digestion and detergent extraction of thyroid membranes, usually from pigs, and is then ready for use, the TSH used as tracer has to meet very high quality requirements. Thus, the radioiodinated TSH must have certain minimum values for the specific binding to TSH receptors (about 30 to 50% specific binding) with correspondingly low values for unspecific binding (not more than 5% of the total activity of the radioactive marker used) in order to fulfil the clinical expectations for a TSH receptor autoantibody assay. This aim has so far been achieved only with considerable complexity, which is described in detail in the above publications of the author Bernard Rees Smith.

In order to obtain a radioiodinated TSH tracer of sufficient quality, the following operations are required:
1. Bovine TSH from bovine pituitaries must be purified by a complicated method. According to the literature, as a rule specific activities of about 30 IU/mg of protein are obtained in the most favourable cases. The best commercially available bovine TSH has a specific activity of 20 IU/mg of protein according to the manufacturer's data (company UCB). However, other bovine TSH preparations are also commercially available, all of which have a lower specific activity.
2. In order to obtain a radioiodinated TSH tracer, this available bovine TSH must be labelled with ¹²⁵I. The iodogenic method, which is complicated but regarded as gentle, is preferably used for this purpose (cf. Bernard Rees Smith in: Methods in Enzymology, Volume 74, pages 408 to 412), incorporation rates of 25 to 70% of ¹²⁵iodine in the protein preparation being obtained. A subsequent purification or separation of the free iodine by gel filtration leads to a total yield of 5-10 x 10⁸ dpm. On the basis of the employed quantity of radioactivity of 1mCi (2.22 x 10⁹ dpm), this corresponds to a radioactivity yield of 22 to 45% in this known tracer production process. However, when used directly in a radioreceptor test, this tracer leads to specific bindings of only about 11% (B₀ - UB) (B₀ = total amount of the bound radioactivity; UB = unspecific binding). In other words, a major part of the radioactivity is bound to TSH derivatives or fragments which have no receptor binding capability. Such specific binding is far from sufficient for the provision of a TSH receptor autoantibody assay which is satisfactory in practice.
3. According to the so far undisputed opinion of the specialists, it is therefore absolutely essential at this stage to subject the tracer obtained by radioiodination of the bovine TSH to a further affinity purification in order to increase the receptor-binding fraction, of the ¹²⁵I-TSH. The purification stage carried out at this stage with the already radioiodinated TSH comprises the following processing steps:
   a) preparation of purified thyroid membranes from the pig (cf. Bernard Rees Smith in Methods in Enzymology),
   b) incubation of the tracer obtained as above with the membrane homogenate, a tracer having a sufficient receptor binding capability being bound to the membrane homogenate,
   c) incubation for 15 minutes at 37°C followed by centrifuging for 15 minutes at 17,000 g in order to obtain a pellet which contains the tracer bound to the membranes,
   d) after removal of the supernatant solution, this pellet must be rehomogenised in a buffer which contains sodium chloride in a concentration of 2M, so that the binding of the TSH tracer to the receptor used for purification is eliminated, and
   e), centrifuging for two hours at 100,000 g, the purified radioiodinated TSH tracers being found in the supernatant solution.

The final step is the removal of the sodium chloride from the tracer solution via a desalination column.

The yield of this extremely inconvenient method is 5%, which leads to a total radioactivity yield in a range from 1.1 to 2.2%. When used in a TSH receptor autoantibody assay, a tracer purified in this manner gives specific binding values of about 45% of the total activity used, which is sufficient for an autoantibody assay. However, the complexity of the method used for the preparation of the radioiodinated TSH tracer results in considerable material losses and also a low radioactivity yield which not only increases the costs but furthermore is disadvantageous for labour and environmental reasons and sets economic limits with regard to the amount of tracer which can be used in an assay and hence to the accuracy of measurement or measuring time.

Endocrinology 1985, Vol. 116, No. 4, pages 1379 and 1382, and the publications cited therein describe the processes for the purification of crude bovine TSH. It is reported that a very pure TSH having extremely high biological activity can be obtained by a reversed-phase HPLC (RPLC) chromatography method described in this publication. On repeating the purification process described in the stated publication, however, it was found that, in accordance with the stated publication, very pure protein fractions can be obtained depending on the chromatographic elution, but that, according to the assay method described below, these fractions have lost their receptor binding capability at least with respect to the porcine TSH receptor described in TSH receptor assays and therefore also cannot be used for the preparation of a TSH tracer having high biological activity.

Further, Endocrinology 1988, Vol. 122, No.1, pages 319 to 324 describes a method of purifying crude bovine TSH in a single step to near homogeneity by affinity chromatography on anti-TSH Sepharose. In order to show the purity of the affinity-purified product, HPLC cation exchange chromatography, including assaying the different fractions for TSH antigen by competitive RIA, and SDS polyacrylamide gel electrophoresis of a radioiodinated product were performed. The biological activity was determined by measuring [³H]thymidine incorporation of FRTL5 cells (a cloned rat thyroid cell line). However, the inventors have found that radioiodination of an affinity-purified TSH preparation leads to a product having only about 9,7% specific binding to a porcine TSH receptor, so that the step of purifying the radioiodinated product in accordance with the teaching of the prior art appears to be necessary as above.

When the work leading to the present invention was begun, the problem to be solved was therefore to find an alternative to the complicated preparation of radioiodinated TSH tracers which to date is regarded as essential among specialists, said alternative permitting the preparation of said tracers in a simpler, more reliable manner with smaller material losses and higher radioactivity yields.

It was found, surprisingly, that this problem can be directly solved if a new TSH preparation is provided which is distinguished with respect to TSH preparations known to date by the fact that its biological activity, measured as specific receptor binding activity, in the assay method described below which is distinguished with respect to TSH preparations known to date by the fact that its biological activity, measured as specific receptor binding activity, in the assay method described below is improved, and that, in its direct radioiodination, it gives a radioiodinated, highly active TSH tracer which can be used directly as a tracer.

According to a first aspect of the present invention, the latter thus provides a process for the production of a radioiodinated TSH tracer which is obtained by radioiodination of a highly purified bovine TSH preparation, which has a biological activity which is improved by a factor of more than two compared with the best commercially available TSH preparations and, with regard to its composition, for example with regard to accompanying substances of the actual biologically active TSH, has a purity such that direct radioiodination by known methods is possible without harmful losses of activity.

Another aspect of the present invention relates to the use of said radioiodinated TSH tracer in TSH radioreceptor autoantibody assays without further purification.

According to a further aspect of the present invention, this novel high-quality radioiodinated TSH tracer, when used in a TSH receptor autoantibody assay operating according to the principles known per se, permits the provision of such an assay in which a considerable reduction in the assay times is possible owing to the increased tracer radioactivity.

These various aspects of the present invention which solve the problems of the prior art are covered by the process Claims 1 to 9 relating to the novel process for the production of a radioiodinated TSH tracer and Claim 10 relating to the use of the obtained TSH tracer in a TSH radioreceptor assay for the determination of TSH receptor autoantibodies.

Further advantageous embodiments and subjects of the invention aspects claimed and described in the present application are evident to one skilled in the art from the description and the embodiments described below.

The invention is based on the surprising finding that, contrary to the prior art, it is possible for the crude (natural) TSH preparations obtained in the conventional manner to be subjected to a certain further purification which results in a considerable increase in the biological activity of the TSH preparations which manifests itself as a receptor binding capability, i.e. an increase by a factor of more than two compared with the best commercial preparations and by very much higher factors compared with commercial preparations having a low biological activity, and that there is no formation of accompanying substances which interfere with the subsequent labelling reactions.

By purifying the TSH preparations and increasing their activity prior to further processing by coupling with labels in order to obtain tracers, in particular prior to radioiodination, it is possible, by direct labelling, to obtain preparations exhibiting high specific binding to the TSH receptor and having a high radioactivity yield without the necessity of the complicated purification of the labelled TSH preparation by affinity chromatography, which has been described to date in the literature as being necessary.

The values obtainable according to the invention for the bound radioactivity, in combination with the good receptor binding capability, make it possible in principle to modify known TSH receptor assays for the determination of TSH autoantibodies in such a way that the measured values required for a statistically relevant result can be obtained in very much shorter times than in all known and commercial processes.

The present invention makes it possible to obtain a radioiodinated bovine TSH which is suitable as a tracer having a radioactivity yield about 40 times higher compared with the prior art, which can be used directly as a tracer in a TSH radioreceptor test after separation of the radioactive iodine not incorporated during the labelling reaction and which has a receptor binding capability which is comparable or even superior to that of the known radioiodinated TSH tracer purified by a complicated procedure.

The TSH preparation according to the invention, obtained by purification of crude bovine TSH, is characterised by a biological activity of more than about 40 IU TSH/mg protein, the biological activity being, as a rule, about 55 IU TSH/mg protein.

The biological activity of the TSH preparation according to the invention is determined by the process below, and the stated values for the biological activity are directly comparable to corresponding values of the known commercial products, since the standard defined below and valid for establishing the international units (IU) is used as a reference substance.

### Determination of the specific receptor binder activity (biological activity) of bovine TSH

In the present description and the Claims, the biological activity is stated in values as obtained in the determination described below:

The biological activity is determined with the aid of the commercially available TRAK-Assay^{R} from Henning Berlin. In this test, ¹²⁵iodine-labelled bovine TSH binds to a porcine TSH receptor.

The TSH preparation whose biological activity is to be determined is used as the sample in the stated assay and competes with the ¹²⁵I-labelled bovine TSH, used as a tracer, for the binding sites of the TSH receptor used in the test. Taking into account the protein concentration of the TSH preparation investigated in each case, TSH preparations having a high specific receptor binding activity lead to the bound amount of labelled TSH tracer or the bound radioactivity being too low.

Using the international standard or using TSH preparations whose biological activity is known in IU TSH/mg protein, the biological activity of unknown TSH preparations can be determined in this manner. The preparations according to the invention are distinguished by biological activities which have more than 40 IU TSH/mg protein and typically about 55 IU TSH/mg protein.

The commercially available TRAK-Assay^{R} used for the determination of biological activity has ¹²⁵I-labelled bovine TSH and porcine TSH receptor as components, the preparation of both components being carried out according to the prior art commented on at the outset and described in the stated publication by Bernard Rees Smith and Hall (Meth. Enzyme. 74, pages 405 to 420, 1981).

The test principle of the known assay may be summarised as follows:

Bovine TSH was extracted in a known manner from bovine pituitaries, concentrated by a chromatographic method, labelled with ¹²⁵iodine and finally subjected to an affinity purification on porcine TSH receptor to obtain the tracer. For the preparation of the porcine TSH receptor, bovine thyroids are homogenised in a detergent-free buffer. Centrifuging is carried out, the supernatant solution with the water-soluble protein is discarded, the resulting pellet is rehomogenised with detergent-containing buffer (the membrane proteins being solubilised), after which centrifuging is carried out a second time, and the resulting supernatant solution is the TSH receptor preparation used for the test.

The TRAK-Assay^{R} is carried out as in the working instructions provided with this test by the manufacturer Henning Berlin, according to the following steps:
1. Pipette a 50 µl sample or standard (TSH preparation to be determined or TSH preparation with known biological activity and concentration, respectively);
2. pipette 50 µl of receptor;
3. incubate for 15 minutes at room temperature;
4. pipette 100 µl of ¹²⁵I-labelled bovine TSH as a tracer (about 12,500 dpm per determination);
5. incubate for 2 hours at room temperature;
6. pipette 2 ml of ice-cold PEG solution (polyethylene glycol 6000, 18%);
7. centrifuge for 20 minutes at 2000 g;
8. decant the resulting supernatant solution;
9. measure the radioactivity in the remaining sediment.

For the determination of the receptor binding activity of a TSH preparation to be tested, the latter is measured in different dilutions. The determination of the protein concentration of the preparation to be tested is carried out in a known manner by the BCA method from the company Pierce. The calibration was carried out by standardisation of the above measuring system with the reference material "International Standard of Thyrotrophin, bovine, code 53/1 of the National Institute of Biological Standards and Control, UK".

For various TSH preparations, the following values were determined:

| TSH preparation (Origin) | | | Biological activity (IU TSH/mg protein) |
|---|---|---|---|
| SIGMA, | Order No.: | T 3538 | 2.0 |
| | Lot No.: | 22H0695 | |
| UCB, | Order No.: | I050BTSH | 20.0 |
| | Batch: | 011-1 | |
| TSH preparation according to the invention which was obtained by the process described below | | | 55.0 |

According to the present invention, TSH preparations having a considerably improved biological activity can thus be obtained. It should be pointed out here that the receptor binding capability, which serves as a measure of the biological activity, is an extremely sensitive product property which must not be confused with an apparent chromatographic homogeneity of a product and which simultaneously represents a very much more sensitive measure than, for example, the antibody binding capability.

A TSH preparation according to the invention is obtainable by subjecting any TSH preparation obtained by the conventional process or any commercial crude bovine TSH preparation first to gentle purification by affinity chromatography on an anti-TSH antibody column and then to ion exchange chromatography over a weakly acidic cation exchanger, all operations being carried out below room temperature and unnecessary waiting times during processing being avoided.

The process for the production of a radioiodinated TSH tracer according to the invention is described in detail below with reference to an embodiment:

### Production of a TSH preparation obtained from crude bovine TSH and having improved biological activity

1. Production of an anti-TSH affinity column for purification of crude, bovine TSH by affinity chromatography
   The production was carried out in accordance with the instructions in the "Carbolink^{R}" Manual from the company Pierce, Rockford/Illinois, USA, whose protein coupling gel was used.
   a) For the preparation of an oxidised anti-TSH antibody, 100 mg of sodium periodate were added to 25 mg of monoclonal anti-TSH antibodies 5405 (Lot No. SP005, obtainable from Oy Medix Biochemica Ab, Kauniainen, Finland) and 25 ml of 0.1 M sodium phosphate buffer (pH 7.0) at room temperature, and mixing was carried out until the solution had become completely clear. Incubation was then carried out for 30 minutes at room temperature.
      The anti-TSH antibody 5405 is specific for hTSH (or the beta subunit of hTSH) and has an affinity constant of 5 x 10⁹ l/mol hTSH. It is prepared in vitro and is sold as an affinity-purified Ig fraction in 0.15 mol/g of NaCl with a content of 0.1% of NaN₃ (protein concentration 1.02 mg/ml). Its use for the purification of bovine TSH is not among the possible uses stated by the manufacturer.
      Instead of the stated antibody, it is possible to use other anti-TSH antibodies whose suitability can be relatively simply determined on the basis of their ability to purify crude bovine TSH by the affinity purification described here.
   (b) The oxidised antibody obtained in stage a) is separated from low molecular weight components by means of desalination columns (NAP-25 from Pharmacia, Uppsala, Sweden). The desalination buffer used is 0.1 M sodium phosphate (pH 7.5).
   (c) After desalination, the antibody (40 ml) is mixed with a Carbolink gel washed beforehand in 0.1 M sodium phosphate (pH 7.0) and is incubated at 4°C for 20 hours with gentle shaking.
   (d) The column material is then transferred to a glass column (BioRad, 1 x 20 cm), washed with 100 ml of 0.1 M glycine HCl buffer (pH 2.8) and equilibrated in 0.1 M sodium phosphate buffer (pH 7.0).
2. Purification of crude, bovine TSH by affinity chromatography
   Various samples of crude, bovine TSH (obtainable from the companies Sigma, Armour, Calbiochem and Fluka) (50 IU in 5 ml of 20 mN sodium phosphate (pH 7.0)) are allowed to seep at 4°C into the anti-TSH affinity column prepared as above and are incubated for 30 minutes. Thereafter, the column is washed with 20 mM sodium phosphate buffer (pH 7.0) (flow rate 1.5 ml/min, total volume 180 ml), after which the elution of bound peptide by means of 50 mM glycine HCl buffer (pH 2.8) is carried out at 1.5 ml/min and 4°C. The column outflow is continuously measured to determine its optical density at 280 nm, and eluting protein is collected (final volume 6 ml). The solution containing the desired protein is immediately diluted 1:2 with water and is used in the subsequent ion exchange chromatography.
   Accompanying investigations during the affinity chromatographic purification described has shown that, under the stated conditions (pH 7.0), bovine TSH is completely bound to the column material. After the column has been washed free of unbound substances, it is desorbed in about 80% yield by elution with the acidic buffer. The results according to the invention were obtained using various crude TSH preparations having very different specific activities (crude TSH from the company Sigma, specific activity 1.8 IU/mg; similar preparations from the companies Calbiochem, Armour and Fluka; and directly freshly prepared bovine pituitary extract).
   Any contaminated preparations can therefore be used for the rapid and effective purification of bovine TSH.
3. Ion exchange chromatography over a weakly acidic cation exchange resin
   The TSH preparation obtained in the purification by affinity chromatography and diluted to twice the volume is applied at 4°C and at a flow rate of 0.8 ml/min directly onto a column equilibrated in 10 mM ammonium acetate (pH 4.5) and containing a weakly acidic cation exchange resin (Euramid WCEX; a weakly acidic cation exchange resin having terminal carboxyl groups and based on a polyamide-coated silica gel). After application, the peptide is eluted in a linear gradient from 10 mM ammonuim acetate (pH 4.5) to 1 M ammonium acetate (pH 4.5) at 0.8 ml/min in 30 min. The column flow is measured continuously for UV absorption at 280 nm. Bovine TSH elutes after about 19 min and is collected directly in a glass vessel cooled with solid carbon dioxide. After freeze-drying of the TSH, the substance can be reconstituted with 20 mM phosphate (pH 7.0) and is then ready for use and can be labelled with ¹²⁵iodine.
   In the ion exchange chromatography, intact TSH is separated from the TSH decomposed into its subunits, which can be effected rapidly, effectively and with high yield under the conditions according to the invention. The purified TSH according to the invention is obtained directly in solution or, after freeze-drying, in dry form, and its biological activity (specific receptor binding capability) is about 55 IU TSH/mg protein.
4. Radioiodination of the TSH preparation according to the invention
   24 µg of the TSH preparation according to the invention and obtained above, in 100 µl of 0.1 M sodium phosphate buffer (pH 7.4), are mixed with 2.5 mCi (25 µl) of ¹²⁵iodine (Amersham), and the reaction is initiated by adding 3 µg of chloramine T (in 20 µl of H₂O). After incubation for 30 s, the reaction mixture is separated directly over a protein Pak-125 column from Waters (Millipore Corporation, USA) (mobile phase: 300 mM sodium phosphate (pH 7.4), 0.1% Lubrol (Sigma), flow rate 0.5 ml/min). The column outflow is measured continuously for radioactivity. Radioiodinated bovine TSH is eluted with an elution time of 17.5 min.
   The yields of the radioiodination are extremely high. Of the 2.5 mCi of ¹²⁵iodine used for the raoioiodination, 1.0 to 1.25 mCi are present in the product, which can be used directly as a ready-to-use tracer. About 50% of the radioactivity used are thus contained in the tracer isolated.
   Instead of radioiodination by the chloramine T method described, radioiodination can also be effected, with essentially the same results, using other known methods, for example the iodogenic method. However, the possibility of carrying out radioiodination without a harmful loss of activity also by the experimentally simple chloramine T method constitutes a considerable practical advantage and reflects the purity of the TSH preparation according to the invention.
5. Use of the radioiodinated TSH tracer in a TSH receptor autoantibody assay
   The radioiodinated bovine TSH isolated is diluted to the desired final total activity in 10 mM Tris-HCl buffer (pH 7.5), 50 mM sodium chloride and 0.1% bovine serum albumin and is used directly as a tracer in a TSH receptor assay.
   In contrast to the unanimous opinion to date in the publications cited at the outset, it was found, surprisingly, that the tracer obtained according to the invention has the required receptor binding capability even without subsequent further purification by affinity chromatography, and was qualitatively comparable with, if not superior to, the tracer produced by the complicated conventional method and subsequently purified by affinity chromatography (cf. Table 1).

**Table 1**

| Comparison of different tracers in the TRAK-Assay® of Henning Berlin | | | |
|---|---|---|---|
| | Best comparative tracer (conventional preparation from TRAK-Assay®) | Tracer produced by the process according to the invention | |
| Total activity (T) | 12,750 dpm | 12,875 dpm | 42,375 dpm |
| Unspecific binding (UB) | 507 dpm | 263 dpm | 1,050 dpm |
| B₀ | 5,625 dpm | 5,493 dpm | 16,950 dpm |
| 5 U/l TSH | 5,363 dpm | 5,024 dpm | 16,100 dpm |
| 15 U/l TSH | 4,620 dpm | 4,376 dpm | 14,913 dpm |
| 45 U/l TSH | 3,300 dpm | 2,916 dpm | 10,675 dpm |
| 135 U/l TSH | 1,866 dpm | 1,556 dpm | 5,463 dpm |
| 405 U/l TSH | 1,209 dpm | 999 dpm | 2,363 dpm |
| UB/T | 4% | 2% | 2.5% |
| (B₀ - UB)/T | 40.1% | 40.7% | 37.5% |
| IC₅₀ | 53 U/l | 47 U/l | 67 U/l |

Owing to the high yields of the process according to the invention, it is possible to compensate a critical deficiency due to the process technology and associated with the preparation of the tracer used to date and its production process:
Owing to the poor yields and high price of the conventional radioiodinated TSH tracer, all known producers of TSH receptor autoantibody assays (apart from the Applicant of the present invention, the companies Byk Sangtec, Biocode and Chronus) are forced to operate with low total radioactivities (10,000 to 15,000 dpm), which, for the users, leads to unfavourably long counting times in the determination of autoantibodies against the TSH receptor (all producers specify counting times of the order of 5 minutes per sample).

Owing to the high biological activity (receptor binding capability) and purity of the TSH preparation obtained in the first step of the present invention, this preparation can be directly radioiodinated without there being a disadvantageous decrease in the specific receptor binding capacity and without it being necessary to carry out a further purification by affinity chromatography in order to obtain a product having sufficient biological activity. In the process according to the invention, a radioiodinated TSH tracer can be prepared without problems even in large amounts, permitting the use of substantially higher quantities of radioactivity in a TSH receptor autoantibody assay and thus resulting in shorter counting times for the user.

Table 2 summarises the differences between the present invention in its various aspects and the prior art.

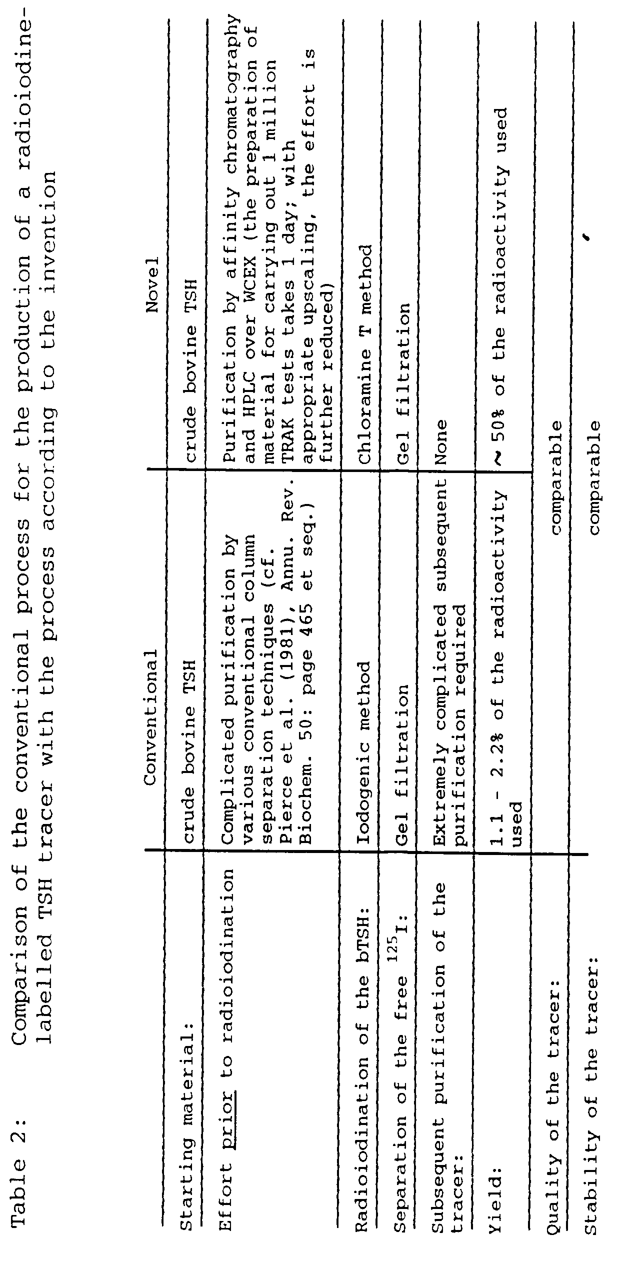

Since it was not known from the prior art that the crude bovine TSH preparations known to date can be purified in a relatively simple manner with recovery of TSH preparations which have a considerably improved biological activity (receptor binding capability) compared with all commercial TSH preparations, and since it was furthermore not known from the prior art that it is possible, by direct radioiodination of any TSH preparation, to obtain a radioiodinated TSH which has the high specific binding properties required for use in a TSH radioreceptor assay, in conjunction with a high radioactivity yield, the present invention constitutes a considerable surprising technical advance.

## Claims

1. Process for the production of a radioiodinated TSH tracer, comprising directly radio-iodinating a TSH preparation having a biological activity of more than 40 IU TSH/mg protein and being obtainable by a process comprising the steps:
(i) treatment of crude bovine TSH over an anti-TSH antibody affinity gel and recovery of an eluate of an intermediate purified TSH preparation, and subsequent
(ii) treatment of said intermediate purified TSH preparation from step (i) by ion exchange chromatography over a weakly acidic cation exchange resin,
(iii) recovery of the fraction of the eluate of step (ii) which absorbs at 280 nm and
(iv) freeze-drying of the eluate fraction obtained from step (iii),
without conducting a further purification of the directly radioiodinated TSH preparation by subsequent affinity chromatography.

2. Process according to Claim 1, characterised in that the purification of the crude bovine TSH by affinity chromatography in step (i) is carried out with the aid of a column containing an anti-TSH antibody affinity gel, which is obtainable by
(a) oxidation of a monoclonal anti-TSH antibody with sodium periodate,
(b) separation of the oxidised antibody from low molecular weight components by means of desalination columns,
(c) incubation of the desalinated oxidised antibody with a protein coupling gel and
(d) transfer of the gel with the bound anti-TSH antibodies to a conventional column,
conventional buffer and washing solutions being used in all steps.

3. Process according to either of Claims 1 or 2, in which the ion exchange chromatography in step (ii) is carried out using a weakly acidic cation exchange resin having terminal carboxyl groups and based on a polyamide-coated silica gel.

4. Process according to any of Claims 1 to 3, in which, as an antibody for the production of the anti-TSH antibody affinity gel for purification step (i), an anti-TSH antibody having binding properties which are identical or similar to those of the monoclonal antibody 5405 (Medix, Kauniainen, Finland) is used .

5. Process according to any of Claims 1 to 4, characterised in that the eluate obtained from step (i) is diluted and is used directly in step (ii), and that the eluate obtained from step (iii) is immediately cooled to the temperature of solid carbon dioxide.

6. Process according to any of Claims 1 to 5, characterized in that said radioiodination is effected by the radioiodination method using chloramine T.

7. Process according to any of Claims 1 to 6, characterised in that a radioiodinated TSH tracer having over 30% specific binding is prepared.

8. Process according to any of Claims 1 to 7, characterised in that the total radioactivity yield of the radioiodination is 45 to 50%.

9. Process according to any of Claims 1 to 8, wherein the biological activity of said TSH preparation to be radioiodinated is 55 IU TSH/mg protein.

10. Use of a radioiodinated TSH tracer obtained by a process according to any of Claims 1 to 9, as a tracer in a TSH radioreceptor assay for the determination of TSH receptor autoantibodies, in which as TSH receptor a porcine TSH receptor preparation is used, in amounts such that the counting times required for the determination of TSH receptor autoantibodies in a biological fluid and applicable to values of B₀ > 12,500 dpm are in the region of 1 min.

## Patentansprüche

1. Verfahren zur Herstellung eines radioiodierten TSH-Tracers, das die direkte Radioiodierung eines TSH-Präparats mit einer biologischen Aktivität von mehr als 40 IU TSH/mg Protein umfaßt, das erhältlich ist durch ein Verfahren, das die Schritte umfaßt:
(i) Behandeln von crudem bovinem TSH an einem Anti-TSH-Aritikörper-Affinitätsgel und Gewinnen eines Eluats eines gereinigten TSH-Präparat-Zwischenprodukts, und anschließende
(ii) Behandeln des gereinigten TSH-Präparat-Zwischenprodukts aus Stufe (i) durch Ionenaustauschchromatographie an einem schwach sauren Kationenaustauscherharz,
(iii) Gewinnen der bei 280 nm absorbierenden Fraktion des Eluats aus Stufe (ii) und
(iv) Gefriertrocknen der Eluatfraktion, die in Stufe (iii) erhalten wurde,
ohne daß eine weitere Reinigung des direkt radioiodierten TSH-Präparats mittels einer nachfolgenden Affinitätschromatographie durchgeführt wird.

2. Verfahren nach Anspruch 1, dadurch gekennzeichnet, daß die Reinigung des cruden bovinen TSH durch Affinitätschromatographie in Stufe (i) mit Hilfe einer Säule durchgeführt wird, die ein Anti-TSH-Antikörper-Affinitätsgel enthält, das erhältlich ist durch
(a) Oxidation eines monoklonalen Anti-TSH-Antikörpers mit Natriumperiodat,
(b) Trennen des oxidierten Antikörpers von niedermolekularen Bestandteilen mit Hilfe von Entsalzungssäulen,
(c) Inkubieren des entsalzten oxidierten Antikörpers mit einem Protein-Kupplungs-Gel und
(d) Überführen des Gels mit den daran gebundenen Anti-TSH-Antikörpern in eine übliche Säule, wobei auf allen Stufen herkömmliche Puffer- und Waschlösungen verwendet werden.

3. Verfahren nach Anspruch 1 oder Anspruch 2, bei dem die Ionenaustauschchromatographie in Stufe (ii) unter Verwendung eines schwach sauren Kationenaustauschharzes mit endständigen Carboxylgruppen auf der Basis eines Polyamidbeschichteten Kieselgels durchgeführt wird.

4. Verfahren nach irgendeinem der Ansprüche 1 bis 3, bei dem man als Antikörper zur Herstellung des Anti-TSH-Antikörper-Affinitätsgels für die Reinigungsstufe (i) einen Anti-TSH-Antikörper mit Bindungseigenschaften verwendet, die denen des monoklonalen Antikörpers 5405 (Medix, Kauniainen, Finnland) gleich oder ähnlich sind.

5. Verfahren nach irgendeinem der Ansprüche 1 bis 4, dadurch gekennzeichnet, daß das aus Stufe (i) erhaltene Eluat verdünnt und unmittelbar in Stufe (ii) eingesetzt wird, und daß das aus Stufe (iii) erhaltene Eluat sofort auf die Temperatur von Trockeneis abgekühlt wird.

6. Verfahren nach irgendeinem der Ansprüche 1 bis 5, dadurch gekennzeichnet, daß man die Radioiodierung als Radioiodierungsverfahren unter Verwendung von Chloramin T durchführt.

7. Verfahren nach irgendeinem der Ansprüche 1 bis 6, dadurch gekennzeichnet, daß ein radioiodierter TSH-Tracer mit einer über 30%igen spezifischen Bindung hergestellt wird.

8. Verfahren nach irgendeinem der Ansprüche 1 bis 7, dadurch gekennzeichnet, daß die gesamte Radioaktivitätsausbeute der Radioiodierung 45 bis 50% beträgt.

9. Verfahren nach irgendeinem der Ansprüche 1 bis 8, bei dem die biologische Aktivität des in die Radioiodierung eingesetzten TSH-Präparats 55 IU TSH/mg Protein beträgt.

10. Verwendung eines radioiodierten TSH-Tracers, der nach einem Verfahren nach irgendeinem der Ansprüche 1 bis 9 erhalten wurde, als Tracer in einem TSH-Radiorezeptor-Assay für die Bestimmung von TSH-Rezeptor-Autoantikörpern, bei dem als TSH-Rezeptor ein porcines TSH-Rezeptor-Präparat verwendet wird, in solchen Mengen, daß die Zählzeiten, die bei der Bestimmung von TSH-Rezeptor-Autoantikörpern in einem biologischen Fluid für Werte B₀ > 12.500 dpm erforderlich sind, im Bereich von 1 Minute liegen.

## Revendications

1. Procédé pour la production d'un traceur de TSH radio-iodée, comportant la radio-iodation directe d'une préparation de TSH présentant une activité biologique supérieure à 40 UI TSH/mg de protéine et pouvant être obtenue par un procédé comportant les étapes consistant à:
(i) traiter de la TSH bovine brute sur un gel présentant une affinité vis-à-vis d'anticorps anti-TSH et récupérer un éluat d'une préparation de TSH purifiée intermédiaire, et ensuite
(ii) traiter ladite préparation de TSH purifiée intermédiaire provenant de l'étape (i) par chromatographie par échange d'ions sur une résine échangeuse d'ions faiblement acide,
(iii) récupérer la fraction de l'éluat de l'étape (ii) qui absorbe à 280 nm, et
(iv) lyophiliser la fraction de l'éluat obtenue dans l'étape (iii),
sans effectuer une purification supplémentaire de la préparation de TSH radio-iodée directement, par une chromatographie par affinité ultérieure.

2. Procédé selon la revendication 1, caractérisé en ce que la purification de la TSH bovine brute par chromatographie par affinité à l'étape (i) est conduite à l'aide d'une colonne contenant un gel présentant une affinité vis-à-vis d'anticorps anti-TSH, qui peut être obtenu par:
(a) oxydation d'un anticorps monoclonal anti-TSH par le periodate de sodium,
(b) séparation de l'anticorps oxydé de composants de faible poids moléculaire, au moyen de colonnes de désalinisation,
(c) incubation de l'anticorps oxydé désalinisé avec un gel de couplage à des protéines, et
(d) transfert du gel avec les anticorps anti-TSH liés sur une colonne classique,
un tampon et des solutions de lavage classiques étant utilisés dans toutes les étapes .

3. Procédé selon l'une des revendications 1 et 2, dans lequel la chromatographie par échange d'ions de l'étape (ii) est conduite en utilisant une résine d'échange de cations faiblement acide présentant des groupes carboxyle terminaux et à base d'un gel de silice revêtu de polyamide.

4. Procédé selon l'une quelconque des revendications 1 à 3, dans lequel, comme anticorps pour la production du gel d'affinité vis-à-vis d'anticorps anti-TSH pour l'étape de purification (i), on utilise un anticorps anti-TSH dont les propriétés de liaison sont identiques ou similaires à celles de l'anticorps monoclonal 5405 (Medix, Kauniainen, Finlande).

5. Procédé selon l'une quelconque des revendications 1 à 4, caractérisé en ce que l'éluat obtenu dans l'étape (i) est dilué et utilisé directement dans l'étape (ii), et en ce que l'éluat obtenu dans l'étape (iii) est refroidi immédiatement à la température du dioxyde de carbone solide.

6. Procédé selon l'une quelconque des revendications 1 à 5, caractérisé en ce que ladite radio-iodation est réalisée par le procédé de radio-iodation utilisant la chloramine T.

7. Procédé selon l'une quelconque des revendications 1 à 6, caractérisé en ce que l'on prépare un traceur de TSH radio-iodée dont la liaison spécifique est supérieure à 30%.

8. Procédé selon l'une quelconque des revendications 1 à 7, caractérisé en ce que le rendement total de radioactivité de la radio-iodation est de 45 à 50%.

9. Procédé selon l'une quelconque des revendications 1 à 8, dans lequel l'activité biologique de ladite préparation de TSH qui doit être radio-iodée est de 55 UI TSH/mg protéine.

10. Utilisation d'un traceur de TSH radio-iodée obtenu par un procédé selon l'une quelconque des revendications 1 à 9 comme traceur dans une radio-détermination de récepteurs de la TSH, pour le dosage d'auto-anticorps dirigés contre les récepteurs de la TSH, dans laquelle on utilise comme récepteurs de la TSH une préparation de récepteurs porcins de la TSH, en quantités telles que les durées de comptage nécessaires pour la détection des autoanticorps de récepteurs de la TSH dans un fluide biologique et applicable à des valeurs de B₀ > 12500 dpm sont dans la région de 1 min.
